# EUROPEAN PATENT APPLICATION

(11) **EP 4 290 529 A1**
(43) Date of publication of application: **13.12.2023**
(21) Application number: 22767435.5
(22) Date of filing: 07.03.2022
(51) Int. Cl.: G16H 50/20, G16H 30/40, G16H 30/20, G16H 50/70, A61B 5/00, A61B 10/02, G06N 20/00, G06Q 30/08

(54) **METHOD FOR TRAINING ARTIFICIAL NEURAL NETWORK HAVING USE FOR DETECTING PROSTATE CANCER FROM TURP PATHOLOGICAL IMAGES, AND COMPUTING SYSTEM PERFORMING SAME**

(30) Priority: 08.03.2021 KR 20210029876
(71) Applicant: Deep Bio Inc., Seoul 08380 (KR)
(72) Inventor: CHO, Joon Young, Seoul 08394 (KR); CHANG, Hye Yoon, Seoul 04153 (KR); KWAK, Tae Yeong, Seoul 05119 (KR); KIM, Sun Woo, Seongnam-si, Gyeonggi-do 13599 (KR)
(74) Representative: Flügel Preissner Schober Seidel
(86) International application number: PCT/KR2022/003178
(87) International publication number: WO 2022/191539

(57) **Abstract**

Disclosed are: a method for training an artificial neural network capable of effectively detecting prostate cancer lesions in TURP pathological images while considering a tissue shape that characteristically appears in a TURP pathological images; and a computing system for performing same. According to one aspect of the present invention, provided is a method for training an artificial neural network having a use for detecting a prostate cancer from a TURP pathological image, comprising steps in which: a neural network training system acquires a plurality of pathological images for primary training, each of the plurality of pathological images for primary training being a prostate needle biopsy pathological image or a radical prostatectomy pathological image; the neural network training system uses the plurality of pathological images for primary training to primarily train an artificial neural network for determining prostate cancer, the artificial neural network for determining prostate cancer being an artificial neural network having a use for detecting prostate cancer from pathological images; the neural network training system acquires a plurality of TURP pathological images; and the neural network training system uses the plurality of TURP pathological images to secondarily train the primarily trained artificial neural network, wherein each of the plurality of TURP pathological images necessarily includes a non-prostate tissue region and/or a cauterized prostate tissue region, and does not include any prostate cancer lesion region.

## Description

### Technical Field

The present disclosure relates to a method of training an artificial neural network for detecting prostate cancer from TURP pathological images and a computing system performing the same. More specifically, the present disclosure relates to a method of training an artificial neural network capable of effectively detecting lesions of prostate cancer in a TURP pathological image while considering tissue morphology typically shown on the TURP pathological image, and a computing system performing the same.

### Background Art

One of main tasks performed by the pathology or pathology department is to perform diagnosis to determine the condition or symptom of a specific disease by reading a patient's biotissue slide image. The existing pathology diagnosis method is performed by visually observing and reading the diagnostic pathologic slide image prepared from a specimen by a pathologist through an optical microscope. It may be the beginning of digital pathology to convert, using a microscope camera connected to a computer, pathologic slides into digital images, and then observe and read the image on a monitor. In recent years, with the advent of digital slide scanners, widespread is a method in which the entire pathologic slides are converted into single digital images, to produce in a form of pathologic slide images, followed by observation and reading through a computer monitor.

Recently, thanks to the development of machine learning, attempts to automate tasks such as recognizing or classifying images by computer systems have been actively made. In particular, attempts have been made to automate the diagnosis that has been performed by skilled medical personnel using a neural network (e.g., a deep learning method using a convolution neural network (CNN)), which is a type of machine learning. Especially, diagnosis through deep learning using the neural network (e.g., CNN) does not simply automate the experience and knowledge of conventionally skilled medical personnel, but in that it finds the characteristic elements through self-learning and derives the desired answer, in some cases, the characteristics of disease factors that the skilled medical personnel were not aware of may be found in images.

In general, the diagnosis of diseases through the neural network using biometric images involves the use of fragments of biometric images (e.g., slide images of biological tissues), that are, patches (also referred to as tiles). In other words, for the patch, the skilled medical professional annotates a state of a specific disease (e.g., whether cancer is developed) and trains the neural network using a plurality of these annotated patches as training data. In this case, a convolution neural network may be used for the neural network.

On the other hand, transurethral resection of prostate (TURP) is often performed for treatment of benign diseases such as prostatic hyperplasia, and it is necessary to pathologically determine the presence or absence of prostate cancer on specimens obtained during the TURP procedure.

It is very difficult to use only TURP pathological imaging data having prostate cancer lesion areas annotated to train a machine learning model that detects prostate cancer from TURP pathological imaging, for the following reasons.

First, the scale of TURP pathological imaging is very broad. An average of N (N is an integer of 2 or more) 2 cm x 3 cm glass slides are produced for each patient, and when they are scanned at 400x and formed into digital imaging, imaging with a size of N x 80,000 x 120,000 pixels is produced. Second, the size of the prostate cancer lesion area within the TURP pathological imaging is very small. Prostate cancer is found in less than 20% of patients undergoing TURP, and even when prostate cancer is detected, it is often detected only in one local area on the pathological imaging. In other words, while the size and capacity of the training data are very broad, the ratio of areas marked as prostate cancer over the total tissue is very small, such that the problem may arise that a lot of data is required to train a machine learning model with sufficient sensitivity using only TURP pathological imaging data.

In addition, the use of specimen pathological imaging data obtained by prostate needle biopsy or radical prostatectomy with the prostate cancer lesion area annotated to train a machine learning model that detects prostate cancer from TURP pathological imaging may be problematic for the following reasons. Due to the nature of TURP, there are non-prostate tissues (bladder and urothelium) or cauterized prostate tissues, which are not shown on specimens undergone with prostate needle biopsy or radical prostatectomy. Therefore, when training the machine learning model that detects prostate cancer from TURP pathological imaging using specimen pathological imaging data obtained by prostate needle biopsy or radical prostatectomy, there may be a problem in that non-prostate tissues (bladder and urothelium) or cauterized prostate tissues are misdiagnosed as cancer.

### Disclosure of the Invention

### Technical Goals

A technical object to be achieved by the present disclosure is to provide a method of training a machine learning model capable of solving the above-mentioned problems and detecting prostate cancer in TURP pathological imaging with high performance. More specifically, an object of the present disclosure is to provide an efficient method to train a machine learning model capable of effectively detecting lesions of prostate cancer in TURP pathological imaging while considering tissue morphology typically shown on the TURP pathological imaging.

### Technical Solutions

According to one aspect of the present disclosure, there is provided a method of training an artificial neural network for detecting prostate cancer from TURP pathological images, including acquiring, by a neural network training system, a plurality of pathological images for primary training, wherein each of the plurality of pathological images for primary training is any one of a prostate needle biopsy pathological image which is a scanned image of slides of a pathological specimen obtained via prostate needle biopsy or a radical prostatectomy pathological image which is a scanned image of slides of a pathological specimen obtained via radical prostatectomy; using, by the neural network training system, the plurality of pathological images for primary training to primarily train the artificial neural network for determining prostate cancer, wherein the artificial neural network for determining prostate cancer is an artificial neural network for detecting prostate cancer from pathological images; acquiring, by the neural network training system, a plurality of TURP pathological images which are scanned images of slides of a pathological specimen obtained via transurethral resection of prostate (TURP); and using, by the neural network training system, the plurality of TURP pathological images to secondarily train the primarily trained artificial neural network, and wherein each of the plurality of TURP pathological images necessarily includes at least one of a non-prostate tissue area or a cauterized prostate tissue area and does not include any prostate cancer lesion area.

In one embodiment, the artificial neural network may be any one of U-Net, DeepLabv3+, Mask R-CNN, and DenseNet.

According to another aspect of the present disclosure, there is provided a method of providing a determination result on a predetermined TURP pathological image to be determined through an artificial neural network trained by the method of training an artificial neural network, including acquiring, by a computing system, the TURP pathological image to be determined, and outputting, by the computing system, a prostate cancer detection result determined by the artificial neural network based on the TURP pathological image to be determined.

According to another aspect of the present disclosure, there is provided a computer program which is installed in a data processing device and recorded on a non-transitory medium for performing the above-described method.

According to another aspect of the present disclosure, there is provided a non-transitory computer-readable recording medium in which a computer program for performing the above-described method is recorded.

According to another aspect of the present disclosure, there is provided a neural network training system which includes a processor and a memory configured to store a computer program, wherein the computer program, when executed by the processor, causes the neural network training system to perform the method of training an artificial neural network described above.

According to another aspect of the disclosure, there is provided a computing system that provides a determination result on a predetermined TURP pathological image to be determined, which includes a processor and a memory configured to store a computer program,
wherein the computer program, when executed by the processor, causes the computing system to perform a method of providing a determination result on the TURP pathological image to be determined through an artificial neural network trained by the artificial neural network training method described above.

According to one aspect of the present disclosure, there is provided a method of training an artificial neural network for detecting prostate cancer from TURP pathological images, including acquiring, by a neural network training system, a plurality of prostate needle biopsy pathological images which are scanned images of slides of a pathological specimen obtained via prostate needle biopsy; acquiring, by the neural network training system, a plurality of radical prostatectomy pathological images which are scanned images of slides of a pathological specimen obtained via radical prostatectomy; using, by the neural network training system, the plurality of prostate needle biopsy pathological images and the plurality of radical prostatectomy pathological images to primarily train an artificial neural network for determining prostate cancer, wherein the artificial neural network for determining prostate cancer is an artificial neural network for detecting prostate cancer from pathological images; acquiring, by the neural network training system, a plurality of TURP pathological images which are scanned images of slides of a pathological specimen obtained via transurethral resection of prostate (TURP); and using, by the neural network training system, the plurality of TURP pathological images to secondarily train the primarily trained artificial neural network, wherein the plurality of TURP pathological images necessarily include at least one of a non-prostate tissue area or a cauterized prostate tissue area and do not include any prostate cancer lesion area.

### Advantageous Effects

According to the technical idea of the present disclosure, it is possible to provide an efficient method and system for training a machine learning model capable of effectively detecting lesions of prostate cancer in TURP pathological images while considering tissue morphology specifically shown on the TURP pathological images.

### Brief Description of Drawings

In order to more fully understand the drawings cited in the detailed description of the present disclosure, a brief description of each drawing is provided.
FIG. 1 is a diagram schematically illustrating an environment in which an artificial neural network training method and a determination result providing method for a pathological specimen in accordance with the technical idea of the present disclosure are performed.
FIG. 2 is a flowchart for explaining a neural network training method in accordance with an embodiment of the present disclosure.
FIG. 3 is an example of a pathological image of a prostate needle biopsy specimen.
FIG. 4A is an example in which a cauterized prostate tissue area is included, and FIG. 4B is an enlarged cauterized prostate tissue area. FIG. 5A shows an example in which a non-prostate tissue area is included, and FIG. 5B shows an enlarged non-prostate tissue area.
FIG. 6 is a diagram illustrating an example of a determination result providing method in accordance with an embodiment of the present disclosure.
FIG. 7 is a diagram illustrating a schematic configuration of an artificial neural network training system in accordance with an embodiment of the present disclosure.
FIG. 8 is a diagram illustrating a schematic configuration of a determination result providing system in accordance with an embodiment of the present disclosure.

### Best Mode for Carrying Out the Invention

Since the present disclosure may apply various transformations and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present disclosure, if it is determined that a detailed description of related known technologies may obscure the gist of the present disclosure, the detailed description will be omitted.

Terms such as first and second may be used to describe various components, but the components should not be limited by the terms. Terms such as first and second do not refer to a particular order and are used only for the purpose of distinguishing one component from another.

The terms used in the present application are used only to describe a particular embodiment and are not intended to limit the present disclosure. Singular expressions include plural expressions unless the context clearly dictates otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail based on embodiments of the present disclosure. Like reference numerals presented in each figure indicate like members.

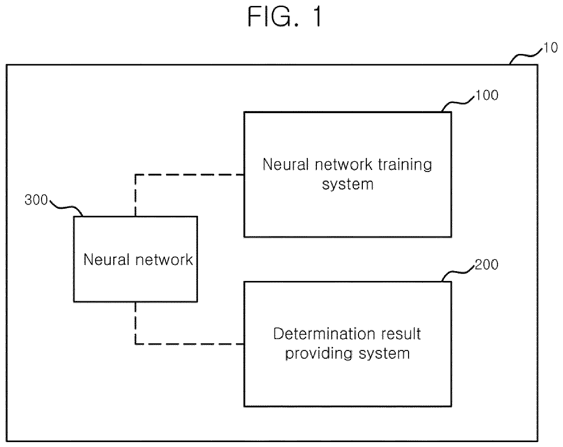

FIG. 1 is a diagram schematically illustrating an environment in which an artificial neural network training method and a determination result providing method for a pathological specimen in accordance with the technical idea of the present disclosure are performed.

Referring to FIG. 1, the artificial neural network training method according to an embodiment of the present disclosure may be performed by a neural network training system 100, and the determination result providing method for a pathological specimen according to an embodiment of the present disclosure may be performed by a determination result providing system for a pathological specimen 200 (hereinafter referred to as a 'determination result providing system').

The artificial neural network training system 100 may train an artificial neural network 300. The artificial neural network 300 may be a neural network for providing diagnostic information for a pathological specimen obtained via transurethral resection of prostate (TURP). A pathologic specimen may be a biopsy taken from various organs of human body and biotissues excised by surgery.

In particular, the artificial neural network 300 may be an artificial neural network to be input with a TURP pathological image to detect prostate cancer from the input TURP pathological image. The determination result providing system 200 may be configured to determine whether prostate cancer is detected from the TURP pathological image using the trained artificial neural network 300. In the present specification, the TURP pathological image may refer to a scanned image of slides of a pathological specimen obtained via transurethral resection of prostate (TURP) or a part thereof. The TURP pathological image may refer to a whole slide image obtained by scanning a pathological slide, but according to an embodiment, it may refer to a patch or tile obtained by dividing the whole slide image of the TURP pathological image into a predetermined unit size.

In one embodiment, the neural network 300 may be a machine learning model trained to output a probability value for whether prostate cancer develops. The neural network 300 may be configured to output a numerical value, that is, a probability value, indicating a determination result (e.g., the probability whether a disease develops) for a target specimen based on the data input through an input layer.

In one embodiment, the artificial neural network 300 may be a machine learning model that is input with a whole slide image to determine whether a lesion exists due to prostate cancer or detect a lesion area, and according to an embodiment, it may be a machine learning model that is input with a patch of a slide image to determine whether a lesion exists due to prostate cancer in the corresponding patch or a lesion area.

In the present specification, the artificial neural network as an artificially constructed neural network based on the operating principle of human neurons may include a multilayer perceptron model and refer to a set of information expressing a series of designs defining an artificial neural network.

In one embodiment, the artificial neural network 300 may be a convolution neural network mainly applied to imaging analysis or include a convolution neural network. In particular, U-Net (Ronneberger2015), DeepLabv3+ (Chen2018), and Mask R-CNN (He2017) which are known to be effective in area division may be used, and if the pixel-by-pixel microscopic area detection function is not required, classification models such as ResNet (He2016) or DenseNet (Huang2017), which has been trained to determine whether the prostate cancer exists in units of small patch images, may be applied.

The neural network training system 100 and/or the determination result providing system 200 may be a computing system that is a data processing device having a computing power for implementing the technical idea of the present disclosure and may generally include a computing device such as a personal computer or a portable terminal as well as a server, which is a data processing device accessible to clients through a network.

An average expert in the art of the present disclosure will be able to easily infer that the neural network training system 100 and/or the determination result providing system 200 may be implemented as any one physical device, but, if necessary, a plurality of physical devices are organically combined to implement the neural network training system 100 and/or the determination result providing system 200 according to the technical idea of the present disclosure.

The neural network training system 100 may train the artificial neural network 300 based on the training data generated from a plurality of pathological specimens. The neural network training system 100 may generate individual training data using scanned images of slides of a pathological specimen or a part (i.e., a patch) of the scanned slide image and input the image to the input layer of the neural network 300 to train the neural network 300. In this case, the pathological specimen may be a specimen obtained by prostate needle biopsy or radical prostatectomy, or a specimen obtained by TURP procedure.

On the other hand, the trained neural network 300 may be stored in the determination result providing system 200, and the determination result providing system 200 may make determination on a predetermined TURP specimen to be diagnosed using the trained artificial neural network.

As shown in FIG. 1, the neural network training system 100 and/or the determination result providing system 200 may be implemented in the form of a subsystem of a predetermined parent system 10. The parent system 10 may be a server. An average expert in the art of the present disclosure will be able to easily infer that the server 10 refers to a data processing device having computing power for implementing the technical ideas of the present disclosure, and in general, any device capable of performing specific services, such as a personal computer and a mobile terminal, as well as a data processing device that is accessible by a client through a network, may be defined as a server.

Alternatively, according to an embodiment, the neural network training system 100 and the determination result providing system 200 may be implemented in a form separated from each other.

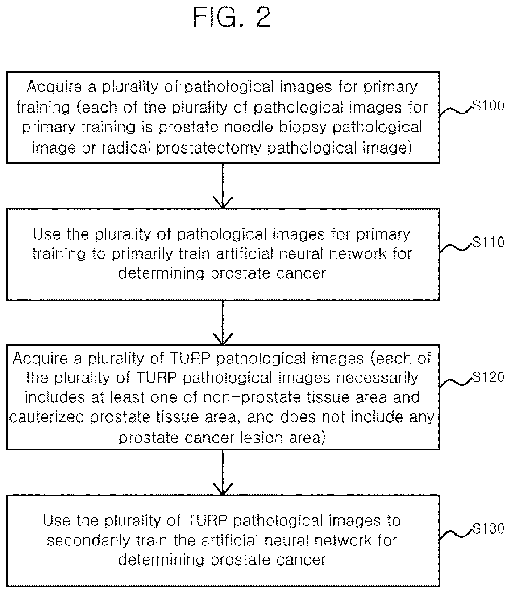

FIG. 2 is a flowchart for explaining the neural network training method in accordance with an embodiment of the present disclosure. The artificial neural network 300 may be trained through the training method of FIG. 2, and as mentioned above, the artificial neural network 300 is an artificial neural network for detecting prostate cancer from pathological images.

Though the artificial neural network 300 is a neural network that performs a function of performing diagnosis on prostate cancer in a TURP pathological image, in the process of training the artificial neural network 300, not only the TURP pathological image but also the image of a specimen obtained by prostate needle biopsy or radical prostatectomy may be used.

Referring to FIG. 2, the neural network training system 100 may acquire a plurality of pathological images for primary training (S 100). Here, each of the plurality of pathological images for primary training may be any one of a prostate needle biopsy pathological image, which is a scanned image of slides of a pathological specimen obtained via prostate needle biopsy, or a radical prostatectomy pathological image, which is a scanned image of slides of a pathological specimen obtained via radical prostatectomy.

On the other hand, each of the plurality of pathological images for primary training may be pre-annotated with lesions due to prostate cancer, and the annotated information may be used as a label of training data.

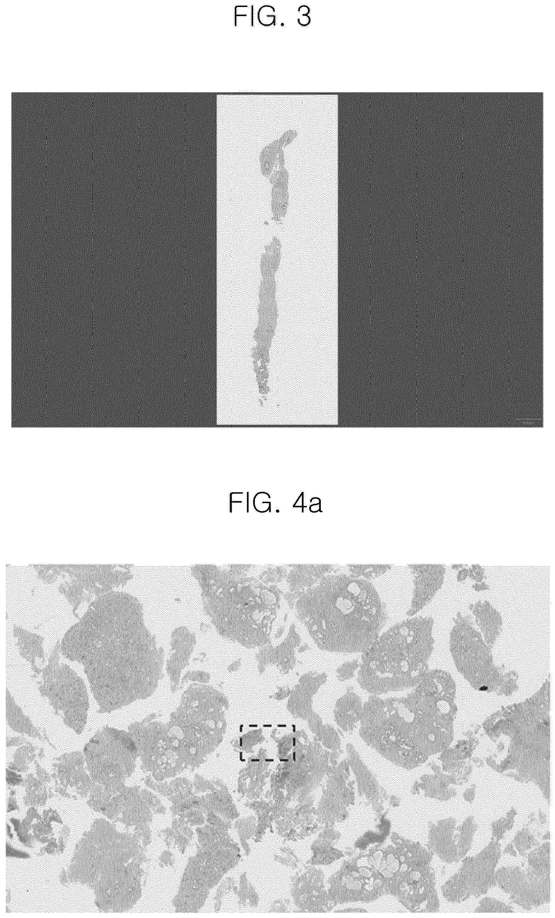

Prostate needle biopsy refers to a method of collecting living tissue of the prostate through needles. An example of a pathological image of a prostate needle biopsy specimen is shown in FIG. 3.

The neural network training system 100 may use the plurality of pathological images for primary training to primarily train the artificial neural network 300 (S 110).

Since the annotated prostate needle biopsy pathological image or the annotated radical prostatectomy pathological image may be obtained relatively easily, in the neural network training method, the artificial neural network 300 is primarily trained using the easily obtainable annotated prostate needle biopsy pathological image or annotated radical prostatectomy pathological image. A certain proportion or more of the plurality of pathological images for primary training may be composed of images including lesion areas due to prostate cancer, thereby increasing the sensitivity of the artificial neural network 300 to a certain level or higher.

On the other hand, in the case of TURP pathological images, due to the nature of the TURP procedure, there is a non-prostate tissue area (bladder and urothelium) or a cauterized prostate tissue area, which does not appear in prostate needle biopsy specimens or radical prostatectomy specimens. Therefore, the following process should be further performed to increase the specificity of the primarily trained artificial neural network 300 for the TURP pathological image.

Referring to FIG. 2 again, the neural network training system 100 may obtain a plurality of TURP pathological images, which are scanned images of slides of a pathological specimen obtained via transurethral resection of prostate (TURP) (S120).

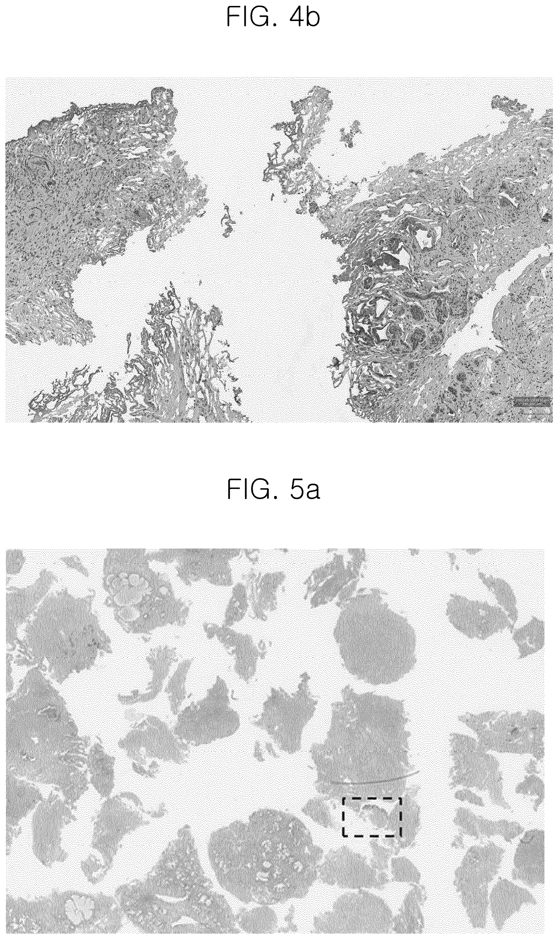

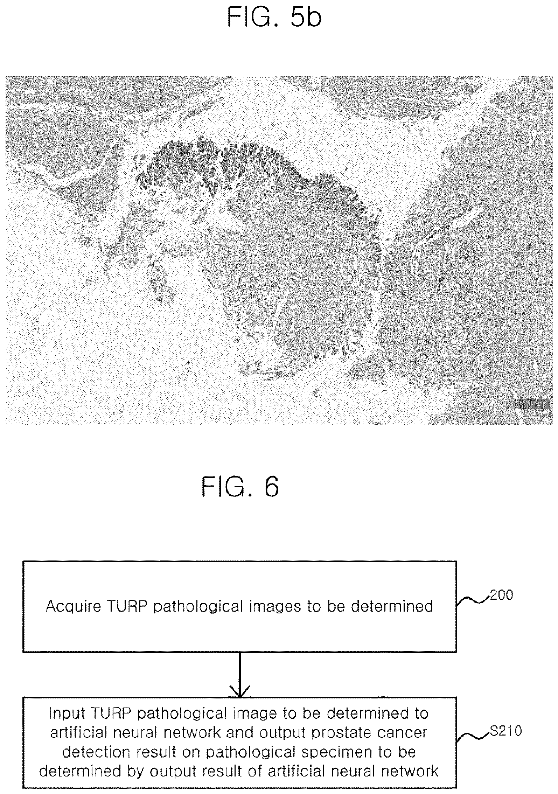

In this case, each of the plurality of TURP pathological images used for secondary training necessarily includes at least one of a non-prostate tissue area or a cauterized prostate tissue area, and does not include any prostate cancer lesion area. FIGS. 4A and 5A are diagrams illustrating examples of TURP pathological images used for secondary training, respectively. FIG. 4A is an example in which a cauterized prostate tissue area is included, and FIG. 4B is an enlarged cauterized prostate tissue area. FIG. 5A is an example in which a non-prostate tissue area is included, and 5B is an enlarged non-prostate tissue area.

In one embodiment, some of the plurality of TURP pathological images used for secondary training may be images that include a non-prostate tissue area but not a prostate cancer lesion area at all, and the remaining part includes a cauterized prostate tissue area but not a prostate cancer lesion area at all.

As such, since all of the plurality of TURP pathological images used for secondary training should not include any prostate cancer lesion area, the plurality of TURP pathological images used for secondary training may be labeled comprehensively as benign and are training data that does not require annotation for a separate lesion area.

Thereafter, the neural network training system 100 may use the plurality of TURP pathological images to secondarily train the primarily trained artificial neural network 300 (S130).

FIG. 6 is a diagram illustrating an example of the determination result providing method in accordance with an embodiment of the present disclosure.

Referring to FIG. 6, the determination result providing system 200 may obtain a predetermined TURP pathological image to be determined (S200). The TURP pathological image to be determined is a scanned image of slides of a predetermined pathological specimen to be determined obtained via transurethral resection of prostate (TURP).

The determination result providing system 200 may input the TURP pathological image to be determined to the artificial neural network 300 and output the prostate cancer determination result determined by the artificial neural network 300 based on the TURP pathological image to be determined (S210).

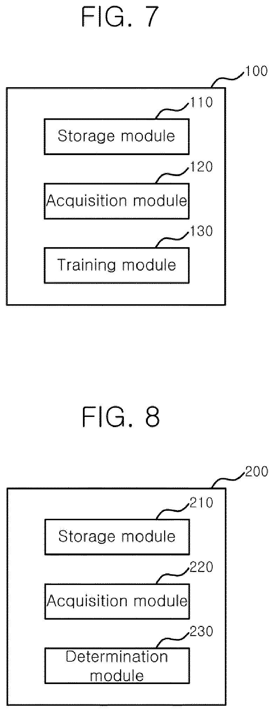

FIG. 7 is a diagram illustrating a schematic configuration of the artificial neural network training system 100 in accordance with an embodiment of the present disclosure, and FIG. 8 is a diagram illustrating a schematic configuration of the determination result providing system 200 in accordance with an embodiment of the present disclosure.

The artificial neural network training system 100 and the determination result providing system 200 may refer to a logical configuration having hardware resources and/or software necessary to implement the technical ideas of the present disclosure, but does not necessarily mean one physical component or one device. In other words, the artificial neural network training system 100 and the determination result providing system 200 may refer to a logical combination of hardware and/or software provided to implement the technical idea of the present disclosure, and if necessary, it may be implemented as a set of logical configurations to implement the technical idea of the present disclosure by being installed in a device spaced apart from each other to perform respective functions. In addition, the artificial neural network training system 100 and the determination result providing system 200 may refer to a set of configurations separately implemented for each function or role to implement the technical idea of the present disclosure. Each configuration of the artificial neural network training system 100 and the determination result providing system 200 may be located on a different physical device or on the same physical device. In addition, according to an embodiment, the combination of software and/or hardware constituting each component of the artificial neural network training system 100 and the determination result providing system 200 may also be located in each different physical device, and configurations located in each different physical device may be organically combined with each other to implement each of the modules.

In addition, the module as used herein may refer to functional and structural combination of hardware to implement the technical ideas of the present disclosure and software to drive the hardware. For example, an average expert in the art of the present disclosure will be able to easily infer that the module may refer to a predetermined code and a logical unit of hardware resources for the predetermined code to be implemented, and not necessarily mean physically connected code, or a type of hardware.

Referring to FIG. 7, the artificial neural network training system 100 may include a storage module 110, an acquisition module 120, and a training module 130. According to an embodiment of the present disclosure, it is apparent that some of the above-described components may not necessarily correspond to components essential to the implementation of the present disclosure, and the artificial neural network training system 100 may include more components according to the embodiment. For example, the artificial neural network training system 100 may further include a communication module (not shown) for communicating with an external device and a control module (not shown) for controlling components and resources of the artificial neural network training system 100.

The storage module 110 may store the artificial neural network 300 to be trained.

The acquisition module 120 may acquire a plurality of pathological images for primary training, and each of the plurality of pathological images for primary training may be any one of a prostate needle biopsy pathological image, which is a scanned image of slides of a pathological specimen obtained via prostate needle biopsy, or a radical prostatectomy pathological image, which is a scanned image of slides of a pathological specimen obtained via radical prostatectomy.

In addition, the acquisition module 120 may acquire a plurality of TURP pathological images, which are scanned images of slides of a pathological specimen obtained via transurethral resection of prostate. In this case, each of the plurality of TURP pathological images may necessarily include at least one of a non-prostate tissue area or a cauterized prostate tissue area, and may not include any prostate cancer lesion area.

The training module 130 may use the plurality of pathological images for primary training to primarily train the artificial neural network for determining prostate cancer, and using the plurality of TURP pathological images, it may secondarily train the primarily trained artificial neural network.

Referring to FIG. 8, the determination result providing system 200 may include a storage module 210, an acquisition module 220, and a determination module 230. According to an embodiment of the present disclosure, some of the above-described components may not necessarily correspond to components essential to the implementation of the present disclosure, and the determination result providing system 200 may include more components according to the embodiment. For example, the determination result providing system 200 may further include a communication module (not shown) and a control module (not shown) for controlling components and resources of the determination result providing system 200.

The storage module 210 may store the trained artificial neural network 300.

The acquisition module 220 may acquire a predetermined TURP pathological image to be determined.

The determination module 230 may input the TURP pathological image to be determined to the artificial neural network 300 and output a detection result on the prostate cancer determined by the artificial neural network 300 based on the TURP pathological image to be determined.

On the other hand, according to an embodiment, the artificial neural network training system 100 and the determination result providing system 200 may include a processor and a memory configured to store a program executed by the processor. The processor may include a single-core CPU or a multi-core CPU. The memory may include a high-speed random access memory and include a non-volatile memory such as one or more magnetic disk storage devices, flash memory devices, or other non-volatile solid-state memory devices. Access to memory by the processor and other components may be controlled by a memory controller.

On the other hand, the method according to an embodiment of the present disclosure may be implemented in the form of a computer-readable program command to be stored on a non-transitory computer-readable recording medium, and a control program and a target program according to an embodiment of the present disclosure may also be stored in a non-transitory computer-readable recording medium. A non-transitory computer-readable recording medium includes all types of recording devices in which data that may be read by a computer system is stored.

Program commands recorded on a recording medium may be specifically designed and configured for the present disclosure or may be known and available to those skilled in the software field.

Examples of a computer-readable recording medium include magnetic media such as hard disks, floppy disks, and magnetic tapes, optical media such as CD-ROMs and DVDs, magneto-optical media such as floptical disks, and hardware devices specifically configured to store and execute program commands such as ROMs, RAM, and flash memory. In addition, the computer-readable recording medium is distributed in computer systems connected through a network, so that computer-readable codes may be stored and executed in a distributed manner.

Examples of program commands include machine codes such as those generated by compilers, as well as devices that electronically process information using interpreters, such as high-level language code that may be executed by a computer.

The above-described hardware device may be configured to operate as one or more software modules to perform the operation of the present disclosure, and vice versa.

The foregoing description of the present disclosure is for illustrative purposes only, and a person skilled in the art to which the present disclosure pertains will be able to understand that it may be easily transformed into other concrete forms without changing the technical idea or essential features of the present disclosure. Therefore, the embodiments described above should be understood as exemplary and not limited in all respects. For example, each component described as a single type may be implemented in a distributed form, and likewise components described as a distributed form may be implemented in a combined form.

The scope of the present disclosure is indicated by the claims to be described later rather than by the above detailed description, and the meaning and scope of the claims and all modifications or modified forms derived from the concept of equivalence thereof should be construed as being included in the scope of the present disclosure.

### Industrial Applicability

The present disclosure may be used for a method of training an artificial neural network for detecting prostate cancer from TURP pathological images and a computing system performing the same.

## Claims

1. A method of training an artificial neural network for detecting prostate cancer from TURP pathological images, the method comprising:
acquiring, by a neural network training system, a plurality of pathological images for primary training, wherein each of the plurality of pathological images for primary training is any one of a prostate needle biopsy pathological image which is a scanned image of slides of a pathological specimen obtained via prostate needle biopsy or a radical prostatectomy pathological image which is a scanned image of slides of a pathological specimen obtained via radical prostatectomy;
using, by the neural network training system, the plurality of pathological images for primary training to primarily train the artificial neural network for determining prostate cancer, wherein the artificial neural network for determining prostate cancer is an artificial neural network for detecting prostate cancer from pathological images;
acquiring, by the neural network training system, a plurality of TURP pathological images which are scanned images of slides of a pathological specimen obtained via transurethral resection of prostate (TURP); and
using, by the neural network training system, the plurality of TURP pathological images to secondarily train the primarily trained artificial neural network,
wherein each of the plurality of TURP pathological images necessarily comprises at least one of a non-prostate tissue area or a cauterized prostate tissue area and does not comprise any prostate cancer lesion area.

2. A method of providing a determination result on a predetermined TURP pathological image to be determined through an artificial neural network trained by the method of training an artificial neural network according to claim 1, comprising:
acquiring, by a computing system, the TURP pathological image to be determined; and
outputting, by the computing system, a prostate cancer detection result determined by the artificial neural network based on the TURP pathological image to be determined.

3. A computer program which is installed in a data processing device and recorded on a non-transitory medium for performing the method according to claim 1 or 2.

4. A non-transitory computer-readable recording medium in which a computer program for performing the method according to claim 1 or 2 is recorded.

5. A computing system, comprising:
a processor; and
a memory configured to store a computer program,
wherein the computer program, when executed by the processor, causes the neural network training system the computing system to perform a method of training an artificial neural network,
wherein the artificial neural network training method comprises:
acquiring a plurality of pathological images for primary training, wherein each of the plurality of pathological images for primary training is any one of a prostate needle biopsy pathological image which is a scanned image of slides of a pathological specimen obtained via prostate needle biopsy or a radical prostatectomy pathological image which is a scanned image of slides of a pathological specimen obtained via radical prostatectomy;
using the plurality of pathological images for primary training to primarily train an artificial neural network for determining prostate cancer, wherein the artificial neural network for determining prostate cancer is an artificial neural network for detecting prostate cancer from pathological images;
acquiring, by the neural network training system, a plurality of TURP pathological images which are scanned images of slides of a pathological specimen obtained via transurethral resection of prostate (TURP); and
using the plurality of TURP pathological images to secondarily train the primarily trained artificial neural network, and
wherein each of the plurality of TURP pathological images necessarily comprises at least one of a non-prostate tissue area or a cauterized prostate tissue area and does not comprise any prostate cancer lesion area.

6. A computing system that provides a determination result on a predetermined TURP pathological image to be determined, comprising:
a processor; and
a memory configured to store a computer program,
wherein the computer program, when executed by the processor, causes the computing system to perform a method of providing a determination result on the TURP pathological image to be determined through an artificial neural network trained by the artificial neural network training method according to claim 1, and
wherein the method of providing the determination result comprises:
acquiring the TURP pathological image to be determined; and
outputting a prostate cancer detection result determined by the artificial neural network based on the TURP pathological image to be determined.
